(19)

Europäisches Patentamt
European Patent Office
Office européen des brevets

(11) **EP 3 669 375 B1**

(12) **EUROPEAN PATENT SPECIFICATION**

(45) Date of publication and mention
of the grant of the patent:
**25.12.2024   Bulletin 2024/52**

(21) Application number: **18783153.2**

(22) Date of filing: **16.08.2018**

(51) International Patent Classification (IPC):
**G16H 50/20** (2018.01)       **G16H 20/13** (2018.01)

(52) Cooperative Patent Classification (CPC):
**G16H 50/20; G16H 20/13**

(86) International application number:
**PCT/US2018/000294**

(87) International publication number:
**WO 2019/036009 (21.02.2019 Gazette 2019/08)**

(54) **SYSTEMS AND METHODS FOR IDENTIFYING HYPERPIGMENTED SPOTS**

SYSTEME UND VERFAHREN ZUR IDENTIFIZIERUNG VON HYPERPIGMENTIERTEN FLECKEN

SYSTÈMES ET PROCÉDÉS D'IDENTIFICATION DE TACHES HYPERPIGMENTÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **18.08.2017   US 201762547196 P**

(43) Date of publication of application:
**24.06.2020   Bulletin 2020/26**

(73) Proprietor: **The Procter & Gamble Company**
**Cincinnati, OH 45202 (US)**

(72) Inventors:
• **PURWAR, Ankur**
  **Singapore 138547 (SG)**
• **KONG, Wai, Kin Adams**
  **Singapore 639798 (SG)**
• **YU, Peicong**
  **Singapore 639798 (SG)**
• **HAKOZAKI, Tomohiro**
  **Cincinnati, OH 45202 (US)**

(74) Representative: **P&G Patent Germany**
**Procter & Gamble Service GmbH**
**Sulzbacher Straße 40**
**65824 Schwalbach am Taunus (DE)**

(56) References cited:
US-A- 6 081 612       US-A1- 2010 185 064
US-A1- 2014 036 054   US-A1- 2016 184 566
US-A1- 2017 231 550

• **GEORGE ZOURIDAKIS ET AL: "Melanoma and Other Skin Lesion Detection Using Smart Handheld Devices", 1 January 2014, MOBILE HEALTH TECHNOLOGIES - BOOK PART III,, PAGE(S) 459 - 496, XP009187194**
• **NURHUDATIANA ARFIKA ET AL: "Automated identification of Relatively Permanent Pigmented or Vascular Skin Marks (RPPVSM)", ICASSP, IEEE INTERNATIONAL CONFERENCE ON ACOUSTICS, SPEECH AND SIGNAL PROCESSING - PROCEEDINGS 1999 IEEE, IEEE, 26 May 2013 (2013-05-26), pages 2984 - 2988, XP032508778, ISSN: 1520-6149, ISBN: 978-0-7803-5041-0, [retrieved on 20131018], DOI: 10.1109/ICASSP.2013.6638205**
• **MACIEL ZORTEA ET AL: "Performance of a dermoscopy-based computer vision system for the diagnosis of pigmented skin lesions compared with visual evaluation by experienced dermatologists", ARTIFICIAL INTELLIGENCE IN MEDICINE, vol. 60, no. 1, 1 January 2014 (2014-01-01), NL, pages 13 - 26, XP055525788, ISSN: 0933-3657, DOI: 10.1016/ j.artmed.2013.11.006**
• **KONSTANTIN KOROTKOV ET AL: "Computerized analysis of pigmented skin lesions: A review", ARTIFICIAL INTELLIGENCE IN MEDICINE, vol. 56, no. 2, 1 October 2012 (2012-10-01), pages 69 - 90, XP055057859, ISSN: 0933-3657, DOI: 10.1016/j.artmed.2012.08.002**

- CHAN FRODO KIN SUN ET AL: "A Study of Distinctiveness of Skin Texture for Forensic Applications Through Comparison With Blood Vessels", IEEE TRANSACTIONS ON INFORMATION FORENSICS AND SECURITY, IEEE, PISCATAWAY, NJ, US, vol. 12, no. 8, 1 August 2017 (2017-08-01), pages 1900 - 1915, XP011648719, ISSN: 1556-6013, [retrieved on 20170509], DOI: 10.1109/TIFS.2017.2692684

- NURHUDATIANA ARFIKA ET AL: "On Criminal Identification in Color Skin Images Using Skin Marks (RPPVSM) and Fusion With Inferred Vein Patterns", IEEE TRANSACTIONS ON INFORMATION FORENSICS AND SECURITY, IEEE, PISCATAWAY, NJ, US, vol. 10, no. 5, 1 May 2015 (2015-05-01), pages 916 - 931, XP011577144, ISSN: 1556-6013, [retrieved on 20150331], DOI: 10.1109/TIFS.2014.2387575

**Description**

FIELD OF THE INVENTION

The present disclosure relates generally to systems for identifying hyperpigmented spots. More specifically, the present disclosure relates to classifying a hyperpigmented spot into one of a plurality of classifications and providing a treatment regimen or product to treat the hyperpigmented spot.

BACKGROUND OF THE INVENTION

Hyperpigmented spots are a common concern in the cosmetic skin industry. Like other perceived cosmetic skin blemishes, hyperpigmented spots can cause emotional and psychological distress to those afflicted by the condition. The vast majority of hyperpigmented facial spots are benign, but in some rare instances, a hyperpigmented spot may be an indication of a more serious skin condition (e.g., melanoma). Although histopathology is commonly used for the diagnosis of skin spots, non-invasive measurements are generally preferred because it reduces or eliminates some of the drawbacks associated with breaking the skin's barrier (risk of infection, scarring, etc.).

Non-invasive diagnostic techniques are known, but some non-invasive diagnostic techniques may not provide the desired level of accuracy for diagnosing spot type and/or severity. For example, different types of hyperpigmented spots can be difficult differentiate using naked eye examination. Additionally, naked eye examination can introduce varying degrees of subjectivity into a skin spot diagnosis, which may result in an inconsistent skin care regimen or skin care product recommendation, especially if different people are consulted for a diagnosis (e.g., beauty consultant versus a dermatologist). Thus, it would be desirable to use a non-invasive diagnostic method that removes at least some, and ideally all, of the subjectivity associated with a naked eye examination.

In some instances, a more objective assessment of hyperpigmentation may be provided by using a colorimeter or spectral meter, but only a small area of skin can be examined at each measurement. As a result, this process requires taking multiple measurements if the number of spots involved is large. In some instances, it can be difficult to provide a desired level of repeatability using colorimeter or spectral meter because it is difficult to relocate the same exact area in each test. Accordingly, a need exists in the industry for a system for identifying and classifying hyperpigmented spots on a subject.

Prior art document US 2014/036054 discloses a system for identifying a hyperpigmented spot, but this system does not use a cross-polarised light, it does not use a baseline image for determining changes in the hyperpigmented spot and performs only a very basic classification thereof.

SUMMARY OF THE INVENTION

Disclosed herein is a system for identifying hyperpigmented spots in accordance with claim 1. The dependent claims relate to further embodiments.

In accordance with the present invention, the system herein includes a computing device that stores logic that, when executed by a processor, causes the computing device to receive a digital image of a subject, where the digital image of the subject is captured using cross-polarized lighting, receive a baseline image of the subject that was not captured using cross-polarized lighting, and identify a hyperpigmented spot in the digital image of the subject. The logic causes the computing device to provide the baseline image and an electronically annotated version the digital image of the subject to distinguish the hyperpigmented spot for display, classify the hyperpigmented spot into a predetermined class, and determine a product for treating the hyperpigmented spot according to the predetermined class. The logic also causes the computing device to provide information related to the product for use by the subject.

Also disclosed is a dispensing device as provided by claim 8.

BRIEF DESCRIPTION OF THE DRAWINGS

FIG. 1 depicts a computing environment for identifying hyperpigmented spots, according to embodiments described herein;
FIG. 2 depicts a user interface for capturing an image of a subject and performing spot determination, according to embodiments described herein;
FIG. 3 depicts a user interface for annotating a hyperpigmented spot, according to embodiments described herein;
FIG. 4 depicts a user interface for creating an ellipse to define a hyperpigmented spot, according to embodiments described herein;

FIG. 5 depicts a user interface for capturing a plurality of hyperpigmented spots on a subject, according to embodiments described herein;

FIG. 6 depicts a user interface for classifying a plurality of different hyperpigmented spots on a subject, according to embodiments described herein;

FIG. 7 depicts a user interface for providing product and treatment recommendations, according to embodiments described herein;

FIG. 8 depicts a flowchart for identifying hyperpigmented spots, according to embodiments described herein; and

FIG. 9 depicts a remote computing device for identifying hyperpigmented spots, according to embodiments described herein.

DETAILED DESCRIPTION OF THE INVENTION

"About" means inclusively within 15% of the stated value.

"Cosmetic" means a non-medical method of providing a desired visual effect on an area of the human body. The visual cosmetic effect may be temporary, semi-permanent, or permanent.

"Cosmetic agent" means any substance, as well any component thereof, intended to be rubbed, poured, sprinkled, sprayed, introduced into, or otherwise applied to a mammalian body or any part thereof to provide a cosmetic effect (e.g., cleansing, beautifying, promoting attractiveness, and/or altering the appearance).

"Cosmetic products" are products that include a cosmetic agent (e.g., skin moisturizers, lotions, perfumes, lipsticks, fingernail polishes, eye and facial makeup preparations, cleansing shampoos, hair colors, shave prep, and deodorants).

"Hyperpigmented" and "hyperpigmented spot" mean a localized portion of skin with relatively high melanin content compared to nearby portions of skin in the same general area of the body. Examples of hyperpigmented spots include, but are not limited to age spots, melasma, chloasma, freckles, post-inflammatory hyperpigmentation, sun-induced pigmented blemishes, and the like.

"Improve the appearance of" means providing a measurable, desirable change or benefit in skin appearance, which may be quantified, for example, by a reduction in the spot area fraction of a hyperpigmented spot and/or an increase in L* value of a hyperpigmented spot. Methods for determining spot area fraction and L* value and changes in these properties are known to those skilled in the art. Some non-limiting examples of these methods are described in co-pending U.S. Serial No. 15/402,332.

"Skin care" means regulating and/or improving a skin condition. Some nonlimiting examples include improving skin appearance and/or feel by providing a smoother, more even appearance and/or feel; increasing the thickness of one or more layers of the skin; improving the elasticity or resiliency of the skin; improving the firmness of the skin; and reducing the oily, shiny, and/or dull appearance of skin, improving the hydration status or moisturization of the skin, improving the appearance of fine lines and/or wrinkles, improving skin exfoliation or desquamation, plumping the skin, improving skin barrier properties, improve skin tone, reducing the appearance of redness or skin blotches, and/or improving the brightness, radiancy, or translucency of skin.

"Subject" refers to a person upon whom the use of methods and systems herein is for cosmetic purposes.

Disclosed herein are systems for identifying hyperpigmented spots and methods of using the system. Different types of hyperpigmented spots have different treatments and prognoses, and thus the systems and methods herein may be configured to provide correct diagnoses and consistent monitoring of hyperpigmented spots for planning management. For example, the systems and method herein may be configured to automatically classify hyperpigmented facial spots into eight different types of hyperpigmentation: solar lentigo, melasma, seborrhoeic keratosis, melanocytic nevus, freckle, actinic keratosis, post inflammatory hyperpigmentation and none of the above. Surprisingly, it has been found that classifying and annotating hyperpigmented spots, as described herein, has had a dramatic effect in proper treatment and reduction of the hyperpigmented spots. It has also been found that the process of creating a fitted ellipse around an image of the hyperpigmented spot and performing pixel analysis to determine texture of a hyperpigmented spot has greatly improved the classification, treatment, and appearance of hyperpigmented spots. In particular, the present method improves the ability of a computer to accurately predict the classification of a hyperpigmented spot.

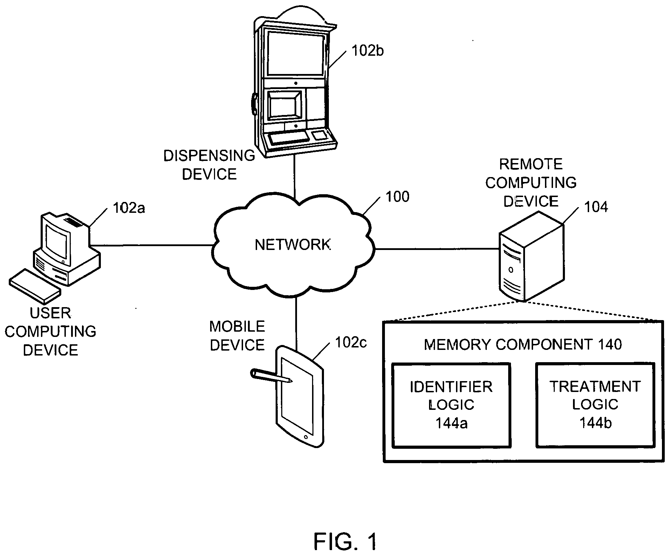

FIG. 1 depicts an exemplary computing environment for identifying hyperpigmented spots. As illustrated, a network 100 is coupled to a user computing device 102a, a dispensing device 102b, a mobile device 102c, and a remote computing device 104. The network 100 may include any wide area network, local network, etc. As an example, the network 100 may include the internet, a public switch telephone network, a cellular network (such as 3G, 4G, LTE, etc.). Similarly, the network 100 may include local networks, such as a local area network, Bluetooth network, Zigbee, near field communication, etc.

Coupled to the network 100 are the user computing device 102a, the dispensing device 102b and the mobile device 102c (individually and collectively referred to herein as "the device 102"). The user computing device 102a may be configured as any computing device that may be utilized for capturing images, communicating with the remote computing device 104, and/or providing one or more user interfaces to a user. As such, the user computing device 102a may be configured as a personal computer, a laptop, and the like. Additionally, while the image capture device may be integrated into the user computing device 102a (and/or the devices 102b, 102c), some embodiments of a system may include a separate image capture device (e.g., a conventional stand-alone digital camera) that captures imagery described herein and is capable of transferring that imagery (or data related to that imagery) to the appropriate device.

The dispensing device 102b may include a computer, display, input device, as well as hardware for dispensing one or more products. As such, the dispensing device 102b may include similar functionality as the user computing device 102a, except with the ability to dispense products, such as one or more cosmetic products or cosmetic agents. The mobile device 102c may also include similar hardware and functionality but may be configured as a mobile phone, tablet, personal digital assistant, and/or the like.

Regardless, the user computing device 102a, the dispensing device 102b, and/or the mobile device 102c may include an image capture device that is configured to capture digital images of a subject. As described in more detail below, some of the images may include a cross polarization light and/or filter. As such, some embodiments of the image capture device may utilize one or more lenses when capturing an image.

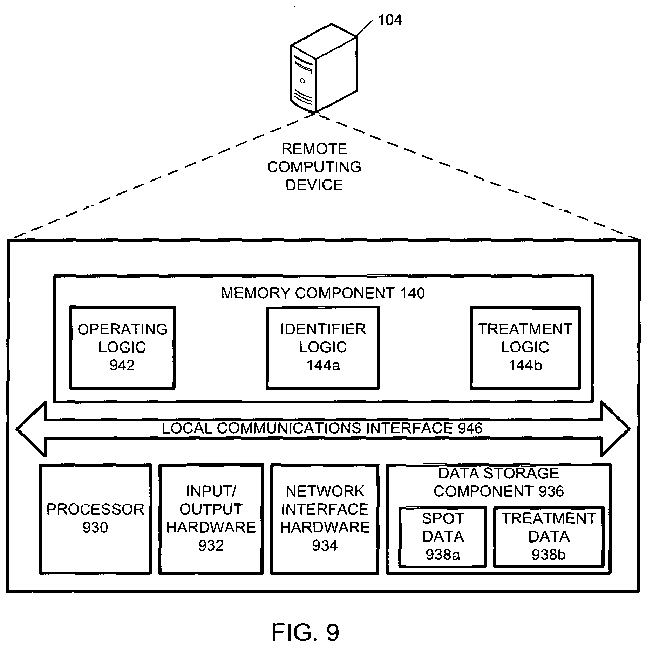

The remote computing device 104 may be configured to communicate with the user computing device 102a, the dispensing device 102b, and/or the mobile device 102c via the network 100. As such, the remote computing device 104 may be configured as a server, personal computer, smart phone, laptop, notebook, kiosk, and the like. The remote computing device 104 may include a memory component 140 and other components depicted in FIG. 9, which store identifier logic 144a and treatment logic 144b. As described in more detail below, the identifier logic 144a may be configured to analyze images to identify a hyperpigmented spot. The treatment logic 144b may be configured to determine one or more product and/or treatment regimens for treating the identified hyperpigmented spot.

It will be understood that while the identifier logic 144a and the treatment logic 144b are depicted as residing in the memory component 140 of the remote computing device 104, this is merely an example. Some embodiments may be configured with logic for performing the described functionality in the user computing device 102a, the dispensing device 102b, and/or the mobile device 102c. Similarly, some embodiments may be configured to utilize another computing device not depicted in FIG. 1 for providing at least a portion of the described functionality.

It will also be understood that the systems in accordance with the present invention are utilized for a consumer in the field of cosmetics (e.g., for skin care). Embodiments related to the medical field include products for and/or methods relating to the treatment of a medical condition and are not part of the present invention. This includes products that require operation by a health care professional; products used by a health care professional in the course of a medical diagnosis; products used in the treatment of a disease or other medical condition requiring treatment by a healthcare professional; products sold with a prescription; and the activities of cosmetic/plastic surgeons, dermatologists, general medical practitioners, and pharmaceutical companies.

Additionally, it will be understood that while the remote computing device 104 is depicted in FIG. 1 as including the logic 144a, 144b, this is also an example. In some embodiments, the device 102 may operate independently from the remote computing device 104 and may only communicate with the remote computing device 104 for updates and other administrative data. Other embodiments may be configured such that the remote computing device 104 provides substantially all of the processing described herein and the user computing device 102a is simply used as a terminal. Still other embodiments may operate as hybrids of these examples and/or leverage one or more of the devices 102 for providing functionality for another of the devices 102. As an example, a user may capture an image via the mobile device 102c and may send that image to the dispensing device 102b to analyze and provide product and treatment recommendations.

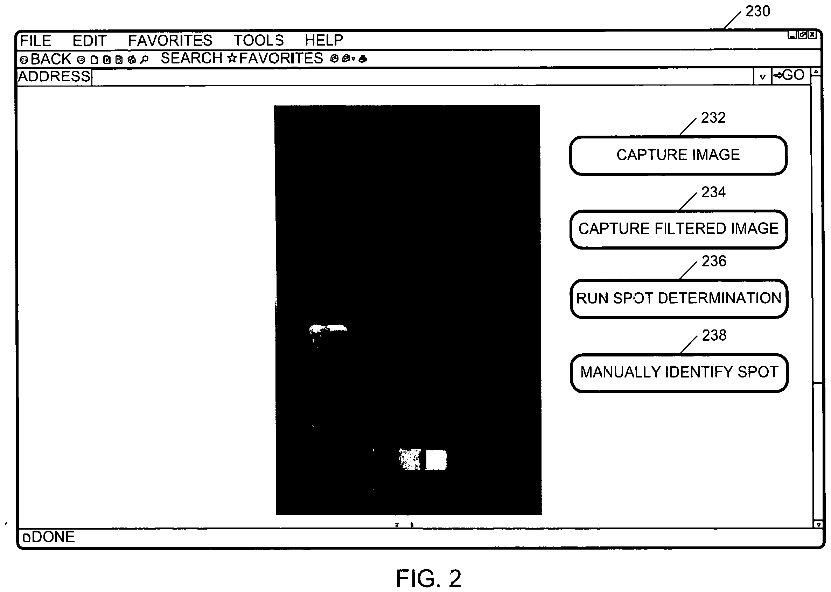

FIG. 2 depicts a user interface 230 for capturing an image of a subject and performing spot determination, according to embodiments described herein. As illustrated, the user interface 230 includes a captured image, a capture image option 232, a capture filtered image option 234, a run spot determination option 236, and a manually identify spot option 238.

In response to selection of the capture image option 232, the device 102 may capture an image of the subject. As discussed above, the image may be captured by the device 102 or may be communicated to the device 102 and/or to the remote computing device 104. Regardless, the image may depict one or more hyperpigmented spots on the face of the subject and may be a white light image, unfiltered image, and/or baseline image of the subject.

In response to selection of the capture filtered image option 234, a cross-polarized image may be captured. Depending on the particular embodiment, the cross-polarized image may be captured using cross-polarized light and/or may be captured via a cross-polarized filter. The cross-polarized image is a digital image in some embodiments. In response to selection of the run spot determination option 236, spot identification and classification may commence. In response to selection of the manually identify spot option 238, the user may manually identify a hyperpigmented spot, as described in more detail below.

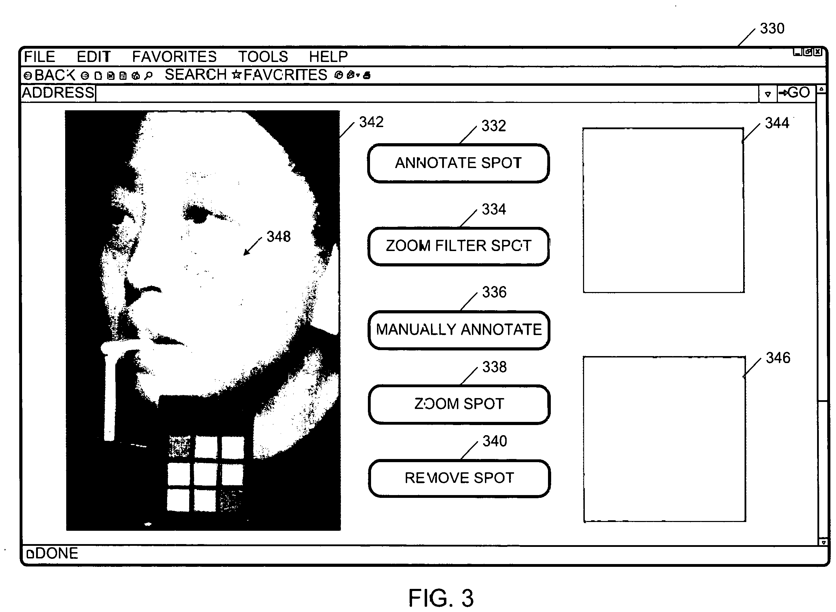

In response to selection of the run spot determination option 236 from FIG. 2, the user interface 330 illustrated in FIG. 3 may be provided. Additionally, the remote computing device 104 (and/or the device 102, depending on the embodiment) may process the image to identify and classify hyperpigmented spots on the image of the subject. The user interface 330 also includes an annotate spot option 332, a zoom filter spot option 334, a manually annotate spot option 336, a zoom spot option 338, and a remove spot option 340.

Also provided in the user interface 330 is an image 342 of the subject, and images of the hyperpigmented spot 344 and 346. In response to selection of the annotate spot option 332, the image 342 may be annotated with an overlay 348 that highlights the identified spot. In response to selection of the zoom filter spot option 334, the digital image of the subject 344 may be provided, which is a cross polarized and zoomed image (e.g., 2x, 3x, 4x, 5x, 10×, or even up to 100x magnification) of the identified spot. In response to selection of the manually annotate spot option 336, additional options may be provided for the user to select and annotate the image manually. In response to selection of the zoom spot option 338, a baseline image 346 may be provided, which is a zoomed image (e.g., 2x, 3x, 4x, 5x, 10x, or even up to 100x magnification) of the annotated hyperpigmented spot (without filter). In some embodiments the digital image of the subject 344 may be compared with the baseline image 346 to determine at least one feature of the hyperpigmented spot. In response to selection of the remove spot option 340, a previously identified spot may be removed from consideration by the user.

It should be understood that while zoomed versions of the images may be compared, as depicted in FIG. 3, this is just one embodiment. Some embodiments are configured to compare a baseline image of a larger portion of a subject's skin, which may contain a plurality of hyperpigmented spots with a filtered image of the same area. Additionally, while some embodiments utilize a baseline image as an unfiltered image and the digital image as the crossfiltered image, this is also an embodiment. Some embodiments compare identical (or substantially similar) images at different points in time to compare progress of a hyperpigmented spot.

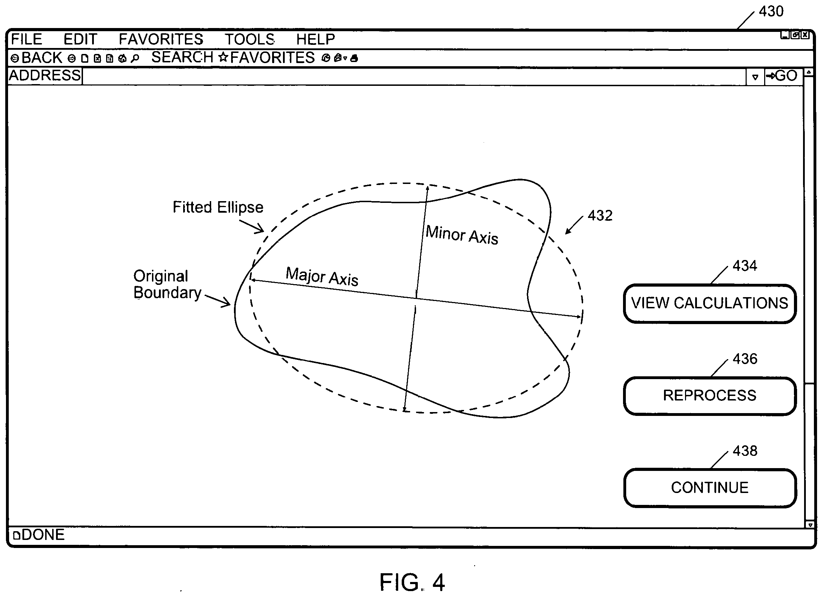

FIG. 4 depicts a user interface 430 for creating a fitted ellipse to define a spatial feature of a hyperpigmented spot, according to embodiments described herein. In response to selection of the annotate spot option 332 from FIG. 3 and/or the run spot determination option 236 from FIG. 2, discolorations in the skin of the subject may be analyzed. As an example, for classification of each spot, 25 dimensional features (or up to about 25) may be derived from a respective region of the cross-polarized image to characterize the hyperpigmented spot. These embodiments take into account the contrast, shape, size, texture, as well as colors in different channels (e.g., RGB color space) for each spot. One or more multiclass learning algorithms may be utilized to classify the spot, including decision tree, AdaBoosting, etc. A multiclass error correcting output code (ECOC) may be utilized as well. The ECOC algorithm is a multiclass classifier built from binary base learners and makes use of code design to distinguish among different classes (i.e., features used to characterize the spot). The ECOC assigns a set of predefined binary codes for each class and a binary base learner is trained for each bit position in the binary code. For a testing sample feature, the classifier will generate a representative binary code, which will be compared with the predefined binary codes for the existing classes. The sample will be assigned to the class having the shortest code distance. An example of features utilized for classification is provided in Table 1, below.

Table 1

| Features | Descriptions |
| --- | --- |
| MeanIn | Mean intensity inside the spot |
| MeanOut | Mean intensity in the neighborhood of the spot |
| Eccentricity | Eccentricity of the fitted ellipse |
| Maj orAxisLength | Major axis length of the fitted ellipse |
| MinorAxisLength | Minor axis length of the fitted ellipse |
| Area | Area of the spot |
| LBPhist_1 | 1st bin value in the LBP histogram |
| LBPhist_2 | 2nd bin value in the LBP histogram |
| LBPhist_3 | 3rd bin value in the LBP histogram |
| LBPhist_4 | 4th bin value in the LBP histogram |
| LBPhist_5 | 5th bin value in the LBP histogram |
| LBPhist_6 | 6th bin value in the LBP histogram |
| LBPhist_7 | 7th bin value in the LBP histogram |
| LBPhist_8 | 8th bin value in the LBP histogram |
| LBPhist_9 | 9th bin value in the LBP histogram |
| LBPhist_10 | 10th bin value in the LBP histogram |
| ColorMaxR | Maximal intensity in R channel |
| ColorMinR | Minimal intensity in R channel |
| ColorMeanR | Mean intensity in R channel |
| ColorMaxG | Maximal intensity in G channel |

(continued)

| Features | Descriptions |
|----------|--------------|
| ColorMinG | Minimal intensity in G channel |
| ColorMeanG | Mean intensity in G channel |
| ColorMaxB | Maximal intensity in B channel |
| ColorMinB | Minimal intensity in B channel |
| ColorMeanB | Mean intensity in B channel |

To derive shape related parameters, a fitted ellipse 432 with the same (or substantially similar) normalized second central moments as the spot region is fitted to the spot boundary, as illustrated in FIG. 4. The fitted ellipse 432 may be utilized to define and/or create a pixel neighborhood for identifying the hyperpigmented spot. Eccentricity of the fitted ellipse may be defined as:

$$e = \sqrt{1 - \frac{b^2}{a^2}}$$

where $a$ is the major axis length and $b$ is the minor axis length. Eccentricity of value 0 indicates a circle while eccentricity of value 1 indicates a line segment.

Texture features of the spot may be derived from the rotational invariant uniform local binary pattern (LBP). The LBP operator assigns a label to every pixel (or a plurality of pixels) of an image by thresholding the $3 \times 3$ pixel neighborhood of each pixel in the image with the central pixel value (as shown in Table 2, below) and mapping the resultant binary pattern. The rotational invariant uniform LBP label is defined as

$$LBP_8^{riu2} = \begin{cases} \sum_{i=1}^{8} s(g_i - g_0) & \text{if } U(LBP_8) \leq 2 \\ 9 & \text{otherwise} \end{cases}$$

where $s(g_i - g_0) = 1$ if $(g_i - g_0) \geq 0$, $s(g_i - g_0) = 0$ if $(g_i - g_0) \leq 0$, and $U(LBP_8)$ is a uniform operator which computes the number of spatial transitions in the pattern (e.g., the bitwise change from 0 to 1 or vice versa). This leads to 10 different labels (0,1,2 ... ,9), whose occurrence is represented as a 10-bin normalised histogram to describe the texture feature of the image. This may be used to measure and/or compare pixel intensity and/or pixel color of a plurality of pixels in the pixel neighborhood, such as depicted in Table 2.

Table 2

| $g_4$ | $g_3$ | $g_2$ |
|-------|-------|-------|
| $g_5$ | $g_0$ | $g_1$ |
| $g_6$ | $g_7$ | $g_8$ |

By using this LBP process, one can determine low level texture features of the hyperpigmented spot. Because textures are often a differentiator in the different types of hyperpigmented spots, this class may be beneficial in identifying a particular type of hyperpigmented spot.

Referring again to FIG. 4, a view calculation option 434 may be provided for viewing the calculations described above. A reprocess option 436 may cause the spot to be reprocessed with the same information, different information, and/or using a different image. Once the features of the hyperpigmented spot are identified, the hyperpigmented spot is classified according to one or more of eight possible classifications: solar lentigo, melasma, seborrhoeic keratosis, melanocytic nevus, freckle, actinic keratosis, post inflammatory hyperpigmentation and none of above. It should be understood that FIG. 4 is depicted as an illustration of calculations and processing that may occur. As such, some embodiments may not actually provide the user interface 430 for display to a user, but may be internally computed for providing the resulting output described herein.

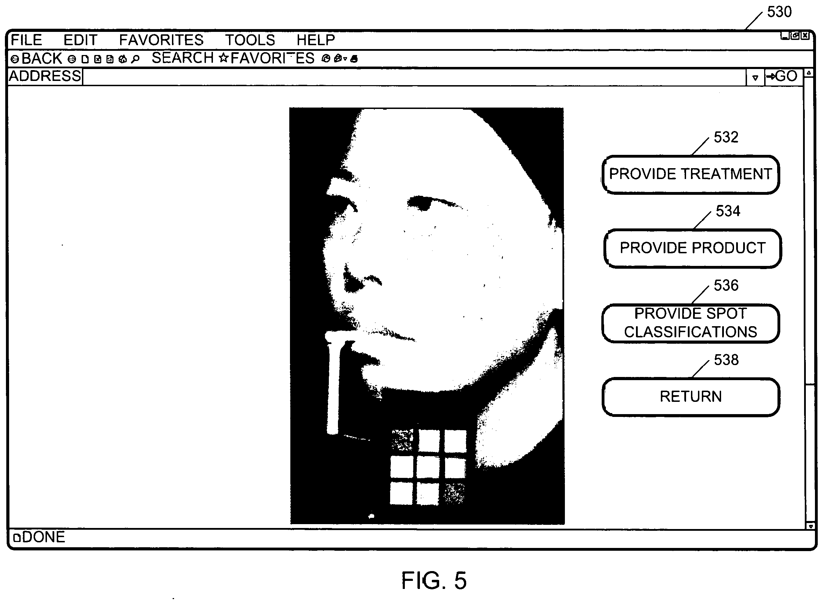

FIG. 5 depicts a user interface 530 for capturing a plurality of hyperpigmented spots on a subject, according to embodiments described herein. As illustrated, a plurality of hyperpigmented spots may be identified and classified on a subject. Additionally, one or more of the identified spots may be annotated to show a user the location and types of spots identified. Further, treatment areas may be annotated on an image of the subject to illustrate where to apply product. FIG. 5 also illustrates a variety of options that a user can select, including a provide treatment option 532, a provide product option 534, a provide spot classifications option 536, and a return option 538.

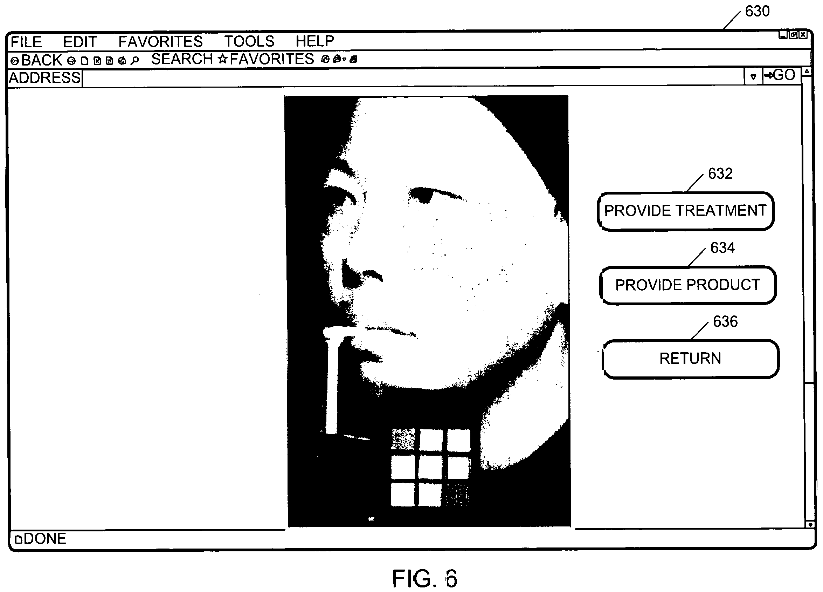

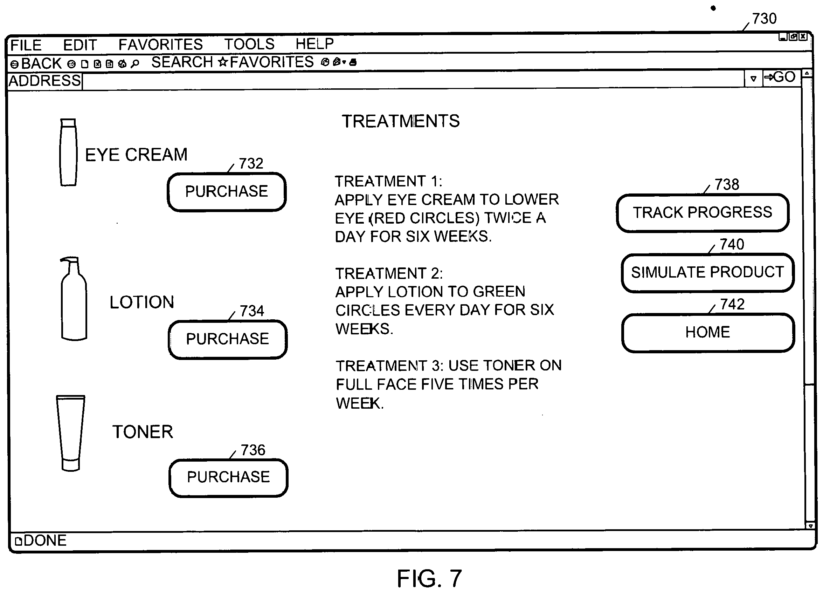

In response to selection of the provide treatment option 532 a treatment regimen may be provided, as illustrated in FIG. 7. In response to selection of the provide product option 534, a product may be provided to the user, as also illustrated in FIG. 7. In response to selection of the provide spot classifications option 536, a listing of classifications for one or more of the hyperpigmented spots may be communicated to a user, for example, via a textual list and/or a color coding (or other coding) on the image to identify a plurality of different classified spots, as illustrated in FIG. 6. In response to selection of the return option 538, the user may be returned to a previous user interface.

FIG. 6 depicts a user interface 630 for classifying a plurality of different hyperpigmented spots on a subject, according to embodiments described herein. In response to classification of the hyperpigmented spots, the user interface 630 provides color coding of those spots for the user to more easily identify the location of each type of spot, as well as identify problem areas and treatment areas. Other options that may be available to a user include a provide treatment option 632, a provide product option 634, and a return option 636.

In response to selection of the provide treatment option 632, a treatment regimen may be provided, as illustrated in FIG. 7. In response to selection of the provide product option 634 a product recommendation may be provided, as also illustrated in FIG. 7.

FIG. 7 depicts a user interface 730 for providing product and treatment recommendations, according to embodiments described herein. In response to a user selection of one or more of the options 532, 534 (FIG. 5) 632, and/or 634 (FIG. 6), the user interface 730 may be provided. As illustrated, the user interface 730 may provide recommended products, as well as purchase options 732, 734, 736 for the user to purchase the product for general skin care and/or for treatment of the types of hyperpigmented spots identified and classified. Depending on the particular embodiment, in response to selection of one or more of the purchase options 732, 734, 736, a product may be dispensed and/or queued for order.

Additionally, a treatment regimen may be provided for one or more of the identified problem areas. The treatment regimen and the recommended products may be based on the classifications of hyperpigmented spots. As one will understand, as the subject may be unable to apply a different product to each individual spot, the product and treatment regimens contemplate that the subject will only be able to apply product to an area of the skin that covers more than one spot. As such, customized treatment regimens and products may be provided to account for this anticipated macro level application of product.

Also provided are a track progress option 738, a simulate product option 740, and a home option 742. In response to selection of the track progress option, the user may view historical images of the subject to illustrate how the hyperpigmented spot has changed over time (either improved with the treatment regimen or regressed without using the treatment regimen). In response to selection of the simulate product option 740, imagery may be provided that simulates improvement that the subject may expect if he/she follows the treatment regimen. In response to selection of the home option 742, the user may be taken to a previous user interface.

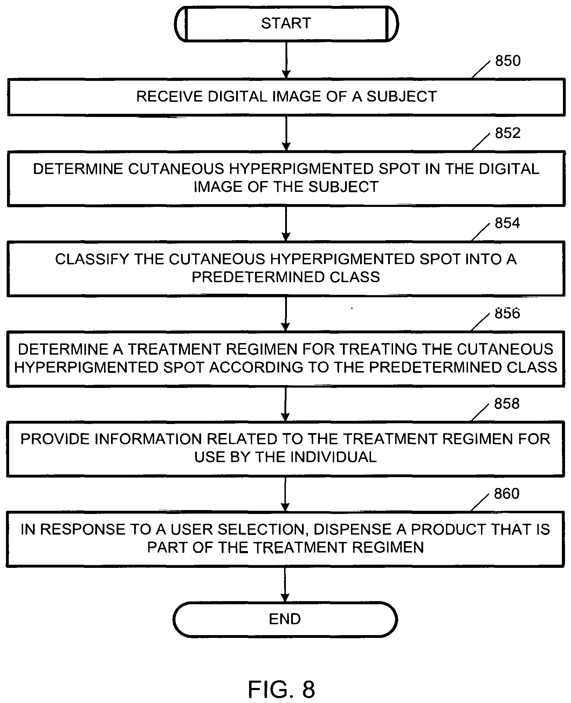

FIG. 8 illustrates a method of identifying hyperpigmented spots, according to embodiments described herein. As illustrated in block 850 of the flowchart 800, a digital image of a subject may be received. In block 852, a hyperpigmented spot may be determined and/or identified in the digital image of the subject. In block 854, the hyperpigmented spot may be classified into a predetermined class. In block 856, a treatment regimen for treating the hyperpigmented spot may be determined, according to the predetermined classification. In block 858, information related to the treatment regimen may be provided for use by the subject. In block 860, in response to a user selection, a product may be dispensed that is part of the treatment regimen. It is to be appreciated that a more detailed description of each step of the method illustrated in FIG. 8 can be found in the preceding disclosure.

FIG. 9 illustrates a remote computing device 104 for identifying hyperpigmented spots, according to embodiments described herein. As illustrated, the remote computing device 104, which includes a processor 930, input/output hardware 932, network interface hardware 934, a data storage component 936 (which stores spot data 938a, treatment data 938b, and/or other data), and the memory component 140. The memory component 140 may be configured as volatile and/or nonvolatile memory and as such, may include random access memory (including SRAM, DRAM, and/or other types of RAM), flash memory, secure digital (SD) memory, registers, compact discs (CD), digital versatile discs (DVD), and/or other types of non-transitory computer-readable mediums. Depending on the particular embodiment, these non-transitory computer-readable mediums may reside within the remote computing device 104 and/or external to the remote computing device 104.

The memory component 140 may store operating logic 942, the identifier logic 144a and the treatment logic 144b. The identifier logic 144a and the treatment logic 144b may each include a plurality of different pieces of logic, each of which may be embodied as a computer program, firmware, and/or hardware, as an example. A local interface 946 is also included in FIG. 9 and may be implemented as a bus or other communication interface to facilitate communication among the components of the remote computing device 104.

The processor 930 may include any processing component operable to receive and execute instructions (such as from a data storage component 936 and/or the memory component 140). The input/output hardware 932 may include and/or be configured to interface with microphones, speakers, a display, and/or other hardware.

The network interface hardware 934 may include and/or be configured for communicating with any wired or wireless networking hardware, including an antenna, a modem, LAN port, wireless fidelity (Wi-Fi) card, WiMax card, Bluetooth chip, USB card, mobile communications hardware, and/or other hardware for communicating with other networks and/or devices. From this connection, communication may be facilitated between the remote computing device 104 and other computing devices, such as the user computing device 102a.

The operating logic 942 may include an operating system and/or other software for managing components of the remote computing device 104. As also discussed above, the identifier logic 144a may reside in the memory component 140 and may be configured to cause the processor 930 to identify, classify, and annotate one or more hyperpigmented spots. Similarly, the treatment logic 144b may be utilized to determine a product and treatment regimen for treating the one or more hyperpigmented spots, as described herein.

It should be understood that while the components in FIG. 9 are illustrated as residing within the remote computing device 104, this is merely an example. In some embodiments, one or more of the components may reside external to the remote computing device 104. It should also be understood that, while the remote computing device 104 is illustrated as a single device, this is also merely an example. In some embodiments, the identifier logic 144a and the treatment logic 144b may reside on different computing devices. As an example, one or more of the functionalities and/or components described herein may be provided by a remote computing device 104 and/or user computing device 102a, which may be coupled to the remote computing device 104 via the network 100.

Additionally, while the remote computing device 104 is illustrated with the identifier logic 144a and the treatment logic 144b as separate logical components, this is also an example. In some embodiments, a single piece of logic may cause the remote computing device 104 to provide the described functionality.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." Additionally, all numeric ranges described herein are inclusive of narrower ranges; delineated upper and lower range limits are interchangeable to create further ranges not explicitly delineated. Embodiments described herein can comprise, consist essentially of, or consist of, the essential components as well as optional pieces described herein. As used in the description and the appended claims, the singular forms "a," "an," and "the" are intended to include the plural forms as well, unless the context clearly indicates otherwise.

**Claims**

1. A system for identifying a hyperpigmented spot for cosmetic purposes, comprising: a computing device that includes a processor and a memory component, wherein the memory component stores logic that, when executed by the processor, causes the computing device to

   (i) receive a digital image of a subject captured using cross-polarized light,
   (ii) receive a baseline image of the subject, where the baseline image is either an unfiltered image of the subject or an image of the subject made at a different point in time,
   (iii) identify a hyperpigmented spot in the image of the subject,
   (iv) compare the baseline image with the image of the subject to determine changes to the hyperpigmented spot;
   (v) electronically annotate the image of the subject to distinguish the hyperpigmented spot in the image,
   (vi) classify the hyperpigmented spot into a predetermined class, the predetermined class includes at least two of the following: solar lentigo, melasma, seborrhoeic keratosis, melanocytic nevus, freckle, actinic keratosis, post inflammatory hyperpigmentation,
   (vii) determine a cosmetic skin care product for treating the hyperpigmented spot according to the predetermined class, and
   (viii) provide information related to the cosmetic skin care product for use by the subject.

2. The system of claim 1, further comprising an image capture device that includes a cross-polarized filter, wherein the image of the subject is captured using the image capture device.

3. The system of any preceding claim, wherein the logic further causes the computing device to determine a textual feature of the hyperpigmented spot from a rotational invariant uniform local binary pattern (LBP).

4. The system of any preceding claim, wherein the logic further causes the computing device to determine a spatial feature of the hyperpigmented spot by creating a fitted ellipse that approximates a shape of the hyperpigmented spot.

5. The system of any preceding claim, wherein the logic further causes the computing device to determine a pixel neighborhood of the hyperpigmented spot; and determine a pixel intensity of a plurality of pixels in the pixel neighborhood.

6. The system of any preceding claim, wherein classifying the hyperpigmented spot includes analyzing between about two and about twenty-five dimensional features of the hyperpigmented spot.

7. The system of claim 6, wherein the dimensional features include at least two dimensional features selected from a mean intensity inside the hyperpigmented spot, a mean intensity in a pixel neighborhood of the hyperpigmented spot, an eccentricity of a fitted ellipse that approximates the hyperpigmented spot, a major axis length of the fitted ellipse, a minor axis length of the fitted ellipse, an area of the hyperpigmented spot, a first bin value in a local binary pattern (LBP) histogram, a second bin value in the LBP histogram, a third bin value in the LBP histogram, a fourth bin value in the LBP histogram, a fifth bin value in the LBP histogram, a sixth bin value in the LBP histogram, a seventh bin value in the LBP histogram, an eighth bin value in the LBP histogram, a ninth bin value in the LBP histogram, a tenth bin value in the LBP histogram, a maximal intensity in an R channel, a minimal intensity in the R channel, a mean intensity in the R channel, a maximal intensity in a G channel, a minimal intensity in the G channel, a mean intensity in the G channel, a maximal intensity in a B channel, a minimal intensity in the B channel, and a mean intensity in the B channel.

8. A dispensing device, comprising:

   a) a cosmetic skin care product;
   b) the system of any one of claims 1 to 7; and
   c) means to dispense said cosmetic skin care product.

**Patentansprüche**

1. System zum Identifizieren eines hyperpigmentierten Flecks für kosmetische Zwecke, umfassend: eine Rechenvorrichtung, die einen Prozessor und eine Speicherkomponente einschließt, wobei die Speicherkomponente eine Logik speichert, die, wenn sie durch den Prozessor ausgeführt wird, die Rechenvorrichtung veranlasst zum

   (i) Empfangen eines digitalen Bilds eines Subjekts, das unter Verwendung von kreuzpolarisiertem Licht erfasst wurde,
   (ii) Empfangen eines Ausgangsbilds des Subjekts, wobei das Ausgangsbild entweder ein ungefiltertes Bild des Subjekts oder ein Bild des Subjekts ist, das zu einem unterschiedlichen Zeitpunkt aufgenommen ist,
   (iii) Identifizieren eines hyperpigmentierten Flecks in dem Bild des Subjekts,
   (iv) Vergleichen des Ausgangsbilds mit dem Bild des Subjekts, um Veränderungen des hyperpigmentierten Flecks zu bestimmen;
   (v) elektronischen Annotieren des Bilds des Subjekts, um den hyperpigmentierten Fleck in dem Bild hervorzuheben,
   (vi) Klassifizieren des hyperpigmentierten Flecks in eine vorherbestimmte Klasse, wobei die vorherbestimmte Klasse mindestens zwei der Folgenden einschließt: Lentigo solaris, Melasma, seborrhoische Keratose, melanocytischer Nävus, Sommersprossen, aktinische Keratose, postinflammatorische Hyperpigmentierung,
   (vii) Bestimmen eines kosmetischen Hautpflegeprodukts für die Behandlung des hyperpigmentierten Flecks gemäß der vorherbestimmten Klasse, und
   (viii) Bereitstellen von Informationen, die sich auf das kosmetische Hautpflegeprodukt beziehen, für die Verwendung durch das Subjekt.

2. System nach Anspruch 1, ferner umfassend eine Bilderfassungsvorrichtung, die einen kreuzpolarisierten Filter einschließt, wobei das Bild des Subjekts unter Verwendung der Bilderfassungsvorrichtung erfasst wird.

3. System nach einem der vorstehenden Ansprüche, wobei die Logik die Rechenvorrichtung ferner veranlasst, ein Texturmerkmal des hyperpigmentierten Flecks aus einem drehinvarianten einheitlichen lokalen Binärmuster (LBP) zu bestimmen.

**4.** System nach einem der vorstehenden Ansprüche, wobei die Logik die Rechenvorrichtung ferner veranlasst, ein räumliches Merkmal des hyperpigmentierten Flecks zu bestimmen, durch Erstellen einer angepassten Ellipse, die sich einer Form des hyperpigmentierten Flecks annähert.

**5.** System nach einem der vorstehenden Ansprüche, wobei die Logik die Rechenvorrichtung ferner veranlasst, eine Pixelumgebung des hyperpigmentierten Flecks zu bestimmen; und Bestimmen einer Pixelintensität einer Vielzahl von Pixeln in der Pixelumgebung.

**6.** System nach einem der vorstehenden Ansprüche, wobei das Klassifizieren des hyperpigmentierten Flecks das Analysieren von etwa zwei bis etwa fünfundzwanzig dimensionalen Merkmalen des hyperpigmentierten Flecks umfasst.

**7.** System nach Anspruch 6, wobei die dimensionalen Merkmale mindestens zwei dimensionale Merkmale einschließen, die ausgewählt sind aus einer mittleren Intensität im Inneren des hyperpigmentierten Flecks, einer mittleren Intensität in einer Pixelumgebung des hyperpigmentierten Flecks, einer Exzentrizität einer angepassten Ellipse, die sich dem hyperpigmentierten Fleck annähert, einer Hauptachsenlänge der angepassten Ellipse, einer Nebenachsenlänge der angepassten Ellipse, einem Bereich des hyperpigmentierten Flecks, einem ersten Binärwert in einem Histogramm eines lokalen binären Musters (LBP), einem zweiten Binärwert in dem LBP-Histogramm, einem dritten Binärwert in dem LBP-Histogramm, einem vierten Binärwert in dem LBP-Histogramm, einem fünften Binärwert in dem LBP-Histogramm, einem sechsten Binärwert in dem LBP-Histogramm, einem siebten Binärwert in dem LBP-Histogramm, einem achten Binärwert in dem LBP-Histogramm, einem neunten Binärwert in dem LBP-Histogramm, einem zehnten Binärwert in dem LBP-Histogramm, einer maximalen Intensität in einem R-Kanal, einer minimalen Intensität in dem R-Kanal, einer mittleren Intensität in dem R-Kanal, einer maximale Intensität in einem G-Kanal, einer minimale Intensität in dem G-Kanal, einer mittlere Intensität in dem G-Kanal, einer maximale Intensität in einem B-Kanal, einer minimale Intensität in dem B-Kanal und einer mittlere Intensität in dem B-Kanal.

**8.** Abgabevorrichtung, umfassend:

   a) ein kosmetisches Hautpflegeprodukt;
   b) das System nach einem der Ansprüche 1 bis 7; und
   c) Mittel zum Abgeben des kosmetischen Hautpflegeprodukts.

**Revendications**

**1.** Système permettant d'identifier une tache hyperpigmentée à des fins cosmétiques, comprenant : un dispositif informatique qui comporte un processeur et un composant de mémoire, dans lequel le composant de mémoire stocke une logique qui, lorsqu'elle est exécutée par le processeur, amène le dispositif informatique à

   (i) recevoir une image numérique d'un sujet capturée à l'aide d'une lumière à polarisation croisée,
   (ii) recevoir une image de référence du sujet, où l'image de référence est soit une image non filtrée du sujet, soit une image du sujet prise à un point différent dans le temps,
   (iii) identifier une tache hyperpigmentée dans l'image du sujet,
   (iv) comparer l'image de référence avec l'image du sujet pour déterminer des changements de la tache hyperpigmentée ;
   (v) annoter électroniquement l'image du sujet pour distinguer la tache hyperpigmentée dans l'image,
   (vi) classer la tache hyperpigmentée dans une classe prédéterminée, la classe prédéterminée comporte au moins deux des éléments suivants : lentigo solaire, mélasma, kératose séborrhéique, naevus mélanocytaire, éphélide, kératose actinique, hyperpigmentation post-inflammatoire,
   (vii) déterminer un produit cosmétique de soin de la peau pour traiter la tache hyperpigmentée selon la classe prédéterminée, et
   (viii) fournir des informations se rapportant au produit cosmétique de soin de la peau utilisable par le sujet.

**2.** Système selon la revendication 1, comprenant en outre un dispositif de capture d'image qui comporte un filtre à polarisation croisée, dans lequel l'image du sujet est capturée à l'aide du dispositif de capture d'image.

**3.** Système selon l'une quelconque revendication précédente, dans lequel la logique amène en outre le dispositif informatique à déterminer une caractéristique textuelle de la tache hyperpigmentée à partir d'un motif binaire local (LBP) uniforme invariant par rapport à la rotation.

4.  Système selon l'une quelconque revendication précédente, dans lequel la logique amène en outre le dispositif informatique à déterminer une caractéristique spatiale de la tache hyperpigmentée par création d'une ellipse ajustée qui se rapproche d'une forme de la tache hyperpigmentée.

5.  Système selon l'une quelconque revendication précédente, dans lequel la logique amène en outre le dispositif informatique à déterminer un voisinage de pixel de la tache hyperpigmentée ; et à déterminer l'intensité d'une pluralité de pixels dans le voisinage de pixel.

6.  Système selon l'une quelconque revendication précédente, dans lequel la classification de la tache hyperpigmentée comporte l'analyse entre environ deux et environ vingt-cinq caractéristiques dimensionnelles de la tache hyperpigmentée.

7.  Système selon la revendication 6, dans lequel les caractéristiques dimensionnelles comportent au moins deux caractéristiques dimensionnelles choisies parmi une intensité moyenne à l'intérieur de la tache hyperpigmentée, une intensité moyenne dans un voisinage de pixel de la tache hyperpigmentée, une excentricité d'une ellipse ajustée qui se rapproche de la tache hyperpigmentée, une longueur de grand axe de l'ellipse ajustée, une longueur de petit axe de l'ellipse ajustée, une aire de la tache hyperpigmentée, une première valeur binaire dans un histogramme de motif binaire local (LBP), une deuxième valeur binaire dans l'histogramme LBP, une troisième valeur binaire dans l'histogramme LBP, une quatrième valeur binaire dans l'histogramme LBP, une cinquième valeur binaire dans l'histogramme LBP, une sixième valeur binaire dans l'histogramme LBP, une septième valeur binaire dans l'histogramme LBP, une huitième valeur binaire dans l'histogramme LBP, une neuvième valeur binaire dans l'histogramme LBP, une dixième valeur binaire dans l'histogramme LBP, une intensité maximale dans un canal R, une intensité minimale dans le canal R, une intensité moyenne dans le canal R, une intensité maximale dans un canal G, une intensité minimale dans le canal G, une intensité moyenne dans le canal G, une intensité maximale dans un canal B, une intensité minimale dans le canal B, et une intensité moyenne dans le canal B.

8.  Dispositif de distribution, comprenant :

    a) un produit cosmétique de soin de la peau ;
    b) le système selon l'une quelconque des revendications 1 à 7 ; et
    c) un moyen de distribuer ledit produit cosmétique de soin de la peau.

FIG. 1

FIG. 2

FIG. 3

FIG. 4

EP 3 669 375 B1

FIG. 5

FIG. 6

FIG. 7

START

RECEIVE DIGITAL IMAGE OF A SUBJECT
850

DETERMINE CUTANEOUS HYPERPIGMENTED SPOT IN THE DIGITAL IMAGE OF THE SUBJECT
852

CLASSIFY THE CUTANEOUS HYPERPIGMENTED SPOT INTO A PREDETERMINED CLASS
854

DETERMINE A TREATMENT REGIMEN FOR TREATING THE CUTANEOUS HYPERPIGMENTED SPOT ACCORDING TO THE PREDETERMINED CLASS
856

PROVIDE INFORMATION RELATED TO THE TREATMENT REGIMEN FOR USE BY THE INDIVIDUAL
858

IN RESPONSE TO A USER SELECTION, DISPENSE A PRODUCT THAT IS PART OF THE TREATMENT REGIMEN
860

END

FIG. 8

REMOTE
COMPUTING
DEVICE

104

MEMORY COMPONENT 140

| OPERATING LOGIC 942 | IDENTIFIER LOGIC 144a | TREATMENT LOGIC 144b |

LOCAL COMMUNICATIONS INTERFACE 946

| PROCESSOR 930 | INPUT/ OUTPUT HARDWARE 932 | NETWORK INTERFACE HARDWARE 934 | DATA STORAGE COMPONENT 936 |

SPOT DATA 938a | TREATMENT DATA 938b

FIG. 9

**EP 3 669 375 B1**

**REFERENCES CITED IN THE DESCRIPTION**

**Patent documents cited in the description**

- US 2014036054 A
- US 402332